# EUROPÄISCHE PATENTSCHRIFT

(11) **EP 2 391 291 B1**
(45) Veröffentlichungstag und Bekanntmachung des Hinweises auf die Patenterteilung: **11.11.2015**
(21) Anmeldenummer: 10700847.6
(22) Anmeldetag: 12.01.2010
(51) Int. Cl.: A61B 19/00

(54) **CHIRURGISCHE REFERENZIERUNGSEINHEIT, CHIRURGISCHES INSTRUMENT UND CHIRURGISCHES NAVIGATIONSSYSTEM**
SURGICAL REFERENCING UNIT, SURGICAL INSTRUMENT, AND SURGICAL NAVIGATION SYSTEM
UNITÉ DE RÉFÉRENCEMENT CHIRURGICAL, INSTRUMENT CHIRURGICAL ET SYSTÈME DE NAVIGATION CHIRURGICALE

(30) Priorität: 27.01.2009 DE 102009007291
(43) Veröffentlichungstag der Anmeldung: 07.12.2011
(73) Patentinhaber: Aesculap AG, 78532 Tuttlingen (DE)
(72) Erfinder: PFEIFER, Tobias, 78662 Bösingen (DE); TOTH, Rainmond, 78573 Wurmlingen (DE)
(74) Vertreter: Hoeger, Stellrecht & Partner Patentanwälte mbB
(86) Internationale Anmeldenummer: PCT/EP2010/050301
(87) Internationale Veröffentlichungsnummer: WO 2010/086219

(56) Entgegenhaltungen:
- WO-A1-2006/012491
- WO-A1-2008/104548
- US-A1- 2004 167 391
- US-A1- 2005 113 659
- US-A1- 2007 016 009

## Beschreibung

Die Erfindung betrifft eine chirurgische Referenzierungseinheit für ein chirurgisches Navigationssystem, mit dem eine Position und/oder Orientierung der Referenzierungseinheit im Raum bestimmbar ist, welche Referenzierungseinheit einen Grundkörper und mindestens ein am Grundkörper angeordnetes Markerelement umfasst, dessen Position im Raum durch das Navigationssystem bestimmbar ist, wobei das mindestens eine Markerelement am Grundkörper beweglich gelagert ist und die Referenzierungseinheit eine erste Schnittstelleneinrichtung umfasst, welche ausgebildet ist zum in Eingriff Bringen mit einer zweiten, an einem chirurgischen Instrument oder einem Implantat vorgesehenen Schnittstelleneinrichtung in einer Verbindungsstellung.

Die Erfindung betrifft ferner ein chirurgisches Instrument oder Implantat mit einer chirurgischen Referenzierungseinheit für ein chirurgisches Navigationssystem, mit welchem Navigationssystem eine Position und/oder Orientierung der Referenzierungseinheit im Raum bestimmbar ist.

Des Weiteren betrifft die Erfindung ein chirurgisches Navigationssystem mit mindestens einer Referenzierungseinheit und mit mindestens einer Nachweisvorrichtung zum Detektieren der Position und/oder Orientierung der Referenzierungseinheit im Raum.

Chirurgische Referenzierungseinheiten für ein chirurgisches Navigationssystem, mit dem eine Position und/oder Orientierung der Referenzierungseinheit im Raum bestimmbar ist, welche Referenzierungseinheiten einen Grundkörper und mindestens ein am Grundkörper angeordnetes Markerelement umfassen, dessen Position im Raum durch das Navigationssystem bestimmbar ist, sind beispielsweise in der US 2006/0173264 A1, der WO 02/01148 A2 sowie der EP 1 498 688 A1 beschrieben. Sie sind üblicherweise derart ausgebildet, dass Relativpositionen zwischen den Markerelementen, insbesondere wenn mehrere Markerelemente vorgesehen sind, nicht verändert werden können. Mit anderen Worten bedeutet dies, dass eine durch die Markerelemente der Referenzierungseinheit vorgegebenen Geometrie oder Markerelementgeometrie nicht geändert werden kann. Da die Referenzierungseinheiten normalerweise mit unterschiedlichen Instrumenten oder Implantaten verbunden werden können, muss vor deren Einsatz eine Kalibrierung des jeweiligen Instruments oder Implantats nach dem Verbinden mit der Referenzierungseinheit vorgenommen werden, um eine eindeutige Zuordnung der Referenzierungseinheit zu dem jeweiligen Instrument oder Implantat zu schaffen und diese Information dem Navigationssystem zu übermitteln.

Eine solche Vorgehensweise ist aufwändig und zudem fehlerbehaftet, da eine Bedienperson bei nicht korrekter Auswahl des mit der Referenzierungseinheit verbundenen Instruments oder Implantats nicht die gewünschten Messwerte durch das Navigationssystem erhalten wird, nämlich eine Position und/oder eine Orientierung des Instruments beziehungsweise Implantats im Raum, bei dem es sich üblicherweise um einen Operationssaal handelt.

Eine chirurgische Referenzierungseinheit der eingangs beschriebenen Art ist aus der US 2007/0016009 A1 bekannt. Weitere Referenzierungseinheiten sind aus der WO 2006/012491 A1, der WO 2008/104548 A1 sowie der US 2005/0113659 A1 bekannt.

Es ist daher Aufgabe der vorliegenden Erfindung, ein chirurgische Referenzierungseinheit, ein chirurgisches Instrument sowie ein chirurgisches Navigationssystem der eingangs beschriebenen Art so zu verbessern, dass die Handhabung derselben vereinfacht und mögliche Fehler bei deren Nutzung vermieden werden.

Diese Aufgabe wird durch eine chirurgische Referenzierungseinheit gemäß Anspruch 1 gelöst.

Eine solche Referenzierungseinheit hat gegenüber den bekannten Referenzierungseinheiten den Vorteil, dass auf Grund der speziellen Schnittstelleneinrichtung vordefinierte Markerelementpositionen des mindestens einen Markerelements automatisch einstellbar sind. Sind mehrere Markerelemente vorgesehen, so können insgesamt durch die erste Schnittstelleneinrichtung beliebige Markerelementkonfigurationen oder Geometrien eingestellt werden, welche beispielsweise einem Instrument eindeutig zuordenbar oder von diesem durch die zweite Schnittstelleneinrichtung vorgebbar sind. Dies vermeidet eine entsprechende, bislang erforderliche Referenzierung oder Kalibrierung des mit der erfindungsgemäßen Referenzierungseinheit verbundenen Instruments oder Implantats, so dass dabei auch keine Fehler passieren können. Mit der ersten Schnittstelleneinrichtung kann so eine beispielsweise am Instrument oder Implantat durch die zweite Schnittstelleneinrichtung vorgegebenen Kodierung auf die Referenzierungseinheit automatisch übertragen werden, so dass mit dem Navigationssystem automatisch zum einen der Typ des Instruments oder Implantats durch Auswerten von Relativpositionen der Markerelemente der Referenzierungseinheit bestimmbar ist und zum anderen eine Position und/oder Orientierung des mit der Referenzierungseinheit verbundenen Instruments oder Implantats beziehungsweise von besonderen Teilen derselben, beispielsweise einer Instrumentenspitze oder eines Implantatschafts.

Günstig ist es, wenn die Referenzierungseinheit mindestens zwei Markerelemente umfasst. Mindestens zwei Markerelemente vorzusehen, gestattet es, eine Relativposition zwischen ihnen zu ändern und abhängig von dieser eine eindeutige Zuordnung zu Instrumenten oder Implantaten zu schaffen.

Besonders vorteilhaft ist es, wenn die Referenzierungseinheit drei, vier, fünf oder sechs Markerelemente umfasst. Je größer die Anzahl der Markerelemente ist, umso genauer kann eine Positions- beziehungsweise Orientierungsbestimmung der Referenzierungseinheit mittels des Navigationssystems erfolgen. Für eine optimale Bestimmung der Positionen und/oder Orientierung der Referenzierungseinheit im Operationssaal werden vorzugsweise drei Markerelemente benötigt. Die Redundanz der ermittelten Daten kann durch Vorsehen zusätzlicher Markerelemente erhöht werden. Ferner kann eine Genauigkeit bei der Bestimmung der Referenzierungseinheit beziehungsweise deren Orientierung oder Position durch Erhöhung der Abstände zwischen den Markerelementen erreicht werden.

Vorzugsweise ist das mindestens eine Markerelement in Form eines aktiven Markerelements ausgebildet. Aktive Markerelemente zu verwenden hat insbesondere bei einer erhöhten Verschmutzungsgefahr des Markerelements Vorteile.

Günstig ist es, wenn das aktive Markerelement eine Sendeeinheit umfasst zum Aussenden elektromagnetischer Strahlung oder von Ultraschall. Ein derartiges Markerelement kann insbesondere auch dann, wenn es etwas verschmutzt ist, Strahlung derart aussenden, dass eine Position des Markerelements von einem Navigationssystem bestimmbar ist.

Die Kosten bei der Herstellung der Referenzierungseinheit lassen sich reduzieren, wenn das mindestens eine Markerelement in Form eines passiven Markerelements ausgebildet ist. Zudem ist für ein passives Markerelement keine Energieversorgungseinrichtung erforderlich.

Damit mit einem Navigationssystem eine Position des passiven Markerelements im Raum einfach und sicher bestimmbar ist, ist es günstig, wenn eine Oberfläche des passiven Markerelements eine elektromagnetische Strahlung oder Ultraschall reflektierende Oberfläche ist. Dabei kann es sich um eine innere oder eine äußere Oberfläche des Markerelements handeln.

Gemäß einer bevorzugten Ausführungsform der Erfindung kann vorgesehen sein, dass die chirurgische Referenzierungseinheit eine erste Kupplungseinrichtung umfasst, welche ausgebildet ist zum Kuppeln mit einer zweiten, entsprechend ausgebildeten Kupplungseinrichtung, welche an einem chirurgischen Instrument oder einem Implantat vorgesehen ist, zum lösbaren Verbinden des Grundkörpers mit dem Instrument oder dem Implantat. Eine solche Kupplungseinrichtung eignet sich insbesondere hervorragend, um die Referenzierungseinheit an einem Instrument oder Implantat mechanisch zu fixieren, insbesondere temporär festzulegen.

Besonders einfach kann die erste Kupplungseinrichtung hergestellt werden, wenn sie mindestens einen ersten Kupplungsvorsprung und/oder mindestens eine erste Kupplungsausnehmung aufweist, welche korrespondierend zu mindestens einer zweiten Kupplungsausnehmung und/oder mindestens einem zweiten Kupplungsvorsprung der zweiten Kupplungseinrichtung ausgebildet sind. So kann auf einfache Weise eine kraft- und/oder formschlüssige Verbindung des mindestens einen ersten Kupplungsvorsprungs und/oder der mindestens einen ersten Kupplungsausnehmung mit korrespondierenden Teilen einer zweiten Kupplungseinrichtung hergestellt werden.

Vorteilhaft ist es, wenn die Referenzierungseinheit eine Rückstelleinrichtung umfasst zum Überführen des mindestens einen Markerelements von einer aus einer Grundstellung, welche das mindestens eine Markerelement einnimmt, wenn die erste Schnittstelleneinrichtung mit einer zweiten Schnittstelleneinrichtung gekoppelt ist, ausgelenkten Markerelementstellung in die Grundstellung zurück. Mit der Rückstelleinrichtung ist es insbesondere möglich, die nicht mit einer zweiten Schnittstelleneinrichtung gekoppelte Referenzierungseinheit in eine definierte Stellung, nämlich die beschriebene Grundstellung zu überführen. Insbesondere kann die Grundstellung so gewählt werden, dass ein einfaches Verbinden der Referenzierungseinheit mit einer zweiten Schnittstelleneinrichtung eines Instruments oder eines Implantats möglich ist.

Die Referenzierungseinheit kann auf einfache Weise definiert in der Grundstellung gehalten werden, wenn die Rückstelleinrichtung mindestens ein Rückstellglied umfasst, welches das mindestens eine Markerelement vorgespannt in der Grundstellung hält.

Vorzugsweise ist das mindestens eine Markerelement aus der Grundstellung entgegen der Wirkung der Rückstelleinrichtung in eine ausgelenkte Markerelementstellung bringbar. Die Rückstelleinrichtung hält somit auch das mindestens eine Markerelement vorgespannt in der ausgelenkten Markerelementstellung. Insbesondere dann, wenn Kraftstöße auf die Referenzierungseinheit einwirken, ermöglicht es die Rückstelleinrichtung auch, das mindestens eine Markerelement wieder in die definierte, ausgelenkte Markerelementstellung zurück zu überführen.

Gemäß einer bevorzugten Ausführungsform der Erfindung kann vorgesehen sein, dass die erste Schnittstelleneinrichtung mindestens ein mit dem mindestens einen Markerelement gekoppeltes Schnittstellenglied umfasst, welches derart angeordnet und beweglich gelagert ist, dass in Folge einer auf das Schnittstellenglied einwirkenden Betätigungskraft das mindestens eine Markerelement von der Grundstellung, welche das mindestens eine Markerelement einnimmt, wenn die erste Schnittstelleneinrichtung mit keiner zweiten Schnittstelleneinrichtung gekoppelt ist, in eine ausgelenkte Markerelementstellung bringbar ist. Das Schnittstellenglied kann also insbesondere als Krafteinleitungselement genutzt werden, um eine Betätigungskraft auf das mindestens eine Markerelement zu übertragen.

Um bei einer Referenzierungseinheit, welche mehrere Markerelemente umfasst, jedes Markerelement individuell betätigen zu können, ist es vorteilhaft, wenn jedem Markerelement ein eigenes Schnittstellenglied zugeordnet ist. Denkbar wäre es auch, nur einer Untermenge der vorgesehenen Markerelemente jeweils ein eigenes Schnittstellenglied zuzuordnen.

Besonders einfach wird der Aufbau der Referenzierungseinheit, wenn das Schnittstellenglied an der ersten Schnittstelleneinrichtung verschiebbar gelagert ist. Denkbar wäre selbstverständlich optional oder alternativ auch eine verschwenkbare Anordnung.

Vorteilhaft ist es, wenn das Schnittstellenglied aus der Grundstellung entgegen der Wirkung der Rückstelleinrichtung in eine ausgelenkte Schnittstellengliedstellung auslenkbar ist. Dies ermöglicht es, das Schnittstellenglied mittels der Rückstelleinrichtung vorgespannt in der ausgelenkten Schnittstellengliedstellung zu halten, wodurch auch das mindestens eine, mit dem Schnittstellenglied gekoppelte Markerelement in einer definierten Position gehalten werden kann.

Damit das Schnittstellenglied einfach und sicher betätigt werden kann, ist es vorteilhaft, wenn es einen in der Grundstellung vom Grundkörper mindestens teilweise abstehenden Betätigungsabschnitt umfasst. Der Betätigungsabschnitt kann vom Grundkörper beispielsweise auch in einer an diesem ausgebildeten Vertiefung vorstehen, so dass der Betätigungsabschnitt über eine insgesamt vom Grundkörper definierte äußere Kontur nicht zwingend vorstehen muss, aber vorstehen kann.

Der Aufbau der Referenzierungseinheit vereinfacht sich weiter, wenn der Grundkörper eine Grundfläche aufweist und wenn der Betätigungsabschnitt in der Grundstellung über die Grundfläche vorsteht. Wird die Grundfläche zum Beispiel eben ausgebildet und der Grundkörper beispielsweise auf eine ebene Grundfläche gelegt, dann wird der Betätigungsabschnitt zwangsweise betätigt und so weit in den Grundkörper hinein verschoben, dass er nicht mehr über dessen ebene Grundfläche vorsteht.

Vorteilhaft ist es, wenn eine Schnittstellengliedführungseinrichtung zum Führen einer Bewegung des Schnittstellenglieds von der Grundstellung in eine ausgelenkte Schnittstellengliedstellung und/oder zurück vorgesehen ist. Die Schnittstellengliedführungseinrichtung ermöglicht es, eine Bewegung des Schnittstellenglieds definiert zu führen.

Besonders einfach lässt sich die Schnittstellengliedführung ausbilden, wenn sie eine Führungshülse umfasst, in welcher das Schnittstellenglied beweglich geführt ist, und wenn ein Betätigungsabschnitt des Schnittstellenglieds in der Grundstellung mindestens teilweise über die Führungshülse vorsteht. Optional kann der Betätigungsabschnitt des Schnittstellenglieds in der Grundstellung auch vollständig von der Führungshülse umgeben sein. Zum Betätigen des Schnittstellenglieds beziehungsweise von dessen Betätigungsabschnitt kann dann beispielsweise mittels eines Vorsprungs in die Führungshülse eingegriffen werden, um das Schnittstellenglied noch weiter in die Führungshülse hinein zu schieben.

Vorzugsweise ist das Schnittstellenglied mit dem mindestens einen Markerelement über ein Kraftübertragungsglied gekoppelt. Mittels des Kraftübertragungsglieds ist es möglich, eine auf den Betätigungsabschnitt des Schnittstellenglieds einwirkende Betätigungskraft auf das mindestens eine Markerelement zu übertragen. Das Kraftübertragungsglied kann insbesondere in Form eines Schub- und Zugglieds ausgebildet sein, um Schub- und/oder Zugkräfte vom Schnittstellenglied auf das mindestens eine Markerelement zu übertragen können.

Vorteilhafterweise sind mindestens zwei Schnittstellenglieder der Referenzierungseinheit unabhängig voneinander bewegbar. Vorzugsweise sind alle Schnittstellenglieder der Referenzierungseinheit unabhängig voneinander bewegbar. Je mehr Schnittstellenglieder unabhängig voneinander bewegbar sind, umso mehr unterschiedliche, jedoch definierte Markerelementgeometrien lassen sich mit der Referenzierungseinheit vorgeben.

Die eingangs gestellte Aufgabe wird bei einem chirurgischen Instrument oder einem Implantat der eingangs beschriebenen Art erfindungsgemäß dadurch gelöst, dass das mindestens eine Markerelement am Grundkörper beweglich gelagert ist, dass die Referenzierungseinheit eine erste Schnittstelleneinrichtung umfasst, welche ausgebildet ist zum in Eingriff Bringen mit einer zweiten, am chirurgischen Instrument oder Implantat vorgesehenen Schnittstelleneinrichtung in einer Verbindungsstellung, und dass das mindestens eine erste Markerelement in Abhängigkeit der in der Verbindungsstellung zusammenwirkenden ersten und zweiten Schnittstelleneinrichtung in eine von der zweiten Schnittstelleneinrichtung vordefinierte Markerelementposition bringbar ist.

Ein solches erfindungsgemäßes chirurgisches Instrument beziehungsweise Implantat ermöglicht es, mit einer der oben beschriebenen, erfindungsgemäßen Standardreferenzierungseinheiten auf Grund der Ausgestaltung insbesondere der zweiten Schnittstelleneinrichtung, welche eine Kodierung für eine Markerelementgeometrie umfassen kann, eine eindeutige Markerelementgeometrie dem Instrument oder Implantat zuzuordnen, um dieses mit dem Navigationssystem eindeutig identifizieren und seine Position und/oder Orientierung im Raum bestimmen zu können. Werden unterschiedliche Instrumente mit unterschiedlichen zweiten Schnittstelleneinrichtungen ausgestattet, dann kann mit lediglich einem Typ einer Referenzierungseinheit jedem Instrument oder Implantat auf Grund des Zusammenwirkens der ersten und zweiten Schnittstelleneinrichtungen in einer Verbindungsstellung eine eindeutige Markerelementgeometrie zugewiesen werden, und zwar automatisch. Ein Kalibrieren des Instruments beziehungsweise eine Referenzierung desselben, um dem gewählten Instrument manuell die Referenzierungseinheit zuzuordnen und initial eine Position und/oder eine Orientierung der Referenzierungseinheit beziehungsweise des Instruments im Raum zu bestimmen, wird überflüssig.

Ferner ist es vorteilhaft, wenn die Referenzierungseinheit eine der oben beschriebenen Referenzierungseinheiten ist. Das chirurgische Instrument zeichnet sich dann durch die bereits oben im Zusammenhang mit den besonderen Ausführungsformen der erfindungsgemäßen Referenzierungseinheiten beschriebenen Vorteile aus.

Günstig ist es, wenn die zweite Schnittstelleneinrichtung mindestens ein Kodierelement umfasst, welches im Zusammenwirken mit der ersten Schnittstelleneinrichtung das mindestens eine Markerelement in die vordefinierte Markerelementposition zwingt. Das mindestens eine Kodierelement ermöglicht es also, das mindestens eine Markerelement in eine vordefinierte Markerelementposition zu zwingen. Damit kann bei mehreren Markerelementen der Referenzierungseinheit eine Markerelementgeometrie mittels des mindestens einen Kodierelements oder mittels mehrerer Kodierelemente der zweiten Schnittstelleneinrichtung gezielt geändert werden, um eine eindeutige Zuordnung der Markerelementgeometrie zu der zweiten Schnittstelleneinrichtung vorzusehen. Die gewünschte Markerelementgeometrie wird also erst beim Zusammenwirken der ersten und zweiten Schnittstelleneinrichtungen vorgegeben und nicht bereits bei der Herstellung der Referenzierungseinheit. Dies ermöglicht es, mit nur einem Grundtyp einer Referenzierungseinheit im Prinzip beliebig viele Markerelementgeometrien einzustellen. Die Markerelementgeometrien können wiederum in einfacher Weise mittels der zweiten Schnittstelleneinrichtung kodiert werden. Somit ist die Kodierung der Referenzierungseinheit durch das Instrument oder das Implantat möglich, wodurch eine initiale oder Startreferenzierung des Instruments überflüssig wird, da auf Grund der im Zusammenwirken der beiden Schnittstelleneinrichtungen eingestellten Markerelementgeometrie bereits eine eindeutige Bestimmung beispielsweise des Typs des Instruments oder Implantats automatisch möglich ist.

Um wahlweise möglichst alle denkbaren Markerelementgeometrien, die mit der Referenzierungseinheit vorgebbar sind, ausnutzen zu können, ist es günstig, wenn die zweite Schnittstelleneinrichtung mindestens so viele Kodierelemente umfasst, wie Markerelemente an der Referenzierungseinheit vorgesehen sind. Ein Kodierelement kann insbesondere auch so ausgebildet sein, dass das mindestens eine Markerelement nicht aus seiner Grundstellung ausgelenkt wird, wenn die erste und zweite Schnittstelleneinrichtung miteinander in Eingriff stehen und zusammenwirken.

Vorteilhaft ist es, wenn das mindestens eine Kodierelement in Form eines Vorsprungs oder einer Ausnehmung ausgebildet ist, mit welchen ein von der ersten Schnittstelleneinrichtung umfasstes Schnittstellenglied in der Verbindungsstellung in Eingriff bringbar ist. In Eingriff Bringen in diesem Sinne bedeutet insbesondere auch, dass sich das mindestens eine Kodierelement und das Schnittstellenglied nur berühren oder aneinander anliegen, wenn sie zusammenwirken. Ein anderweitiges kraft- und/oder formschlüssiges in Eingriff Bringen ist nicht zwingend erforderlich, optional jedoch möglich.

Besonders einfach lassen sich Markerelementgeometrien einstellen, wenn die Markerelementposition durch eine Tiefe der Ausnehmung oder eine Höhe des Vorsprungs vorgebbar ist. So kann auf einfache Weise und rein mechanisch eine Markerelementposition durch das mindestens eine Kodierelement vorgegeben werden.

Die Konstruktion der zweiten Schnittstelleneinrichtung wird besonders einfach, wenn sie einen zweiten Grundkörper umfasst, welcher das mindestens eine Kodierelement trägt. Ein weiterer Grundkörper der zweiten Schnittstelleneinrichtung, ebenso wie ein Grundkörper der ersten Schnittstelleneinrichtung ist allgemein jeweils auch im Sinne eines Trägers oder einer Trägereinheit für das mindestens eine Markerelement beziehungsweise das mindestens eine Kodierelement zu verstehen.

Die Herstellung der Schnittstelleneinrichtung vereinfacht sich weiter, wenn das mindestens eine Kodierelement unbeweglich an der zweiten Schnittstelleneinrichtung ausgebildet ist. Die zweite Schnittstelleneinrichtung kann so insbesondere ohne jegliche beweglichen Teile ausgebildet werden.

Um das Instrument auf einfache Weise mit der Referenzierungseinheit verbinden zu können, ist es günstig, wenn es eine zweite Kupplungseinrichtung umfasst, welche ausgebildet ist zum Kuppeln mit einer ersten, entsprechend ausgebildeten Kupplungseinrichtung, welche an der Referenzierungseinheit vorgesehen ist, zum lösbaren Verbinden des Grundkörper mit dem Instrument oder Implantat.

Günstig ist es, wenn die zweite Kupplungseinrichtung mindestens einen zweiten Kupplungsvorsprung und/oder mindestens eine zweite Kupplungsausnehmung aufweist, welche korrespondierend zu mindestens einer ersten Kupplungsausnehmung und/oder mindestens einem ersten Kupplungsvorsprung der ersten Kupplungseinrichtung ausgebildet sind. Insbesondere kann so auf einfache Weise eine kraft- und/oder formschlüssige Verbindung zwischen dem Instrument oder Implantat einerseits und der Referenzierungseinheit andererseits in einer Kupplungs- oder Verbindungsstellung hergestellt werden.

Die eingangs gestellte Aufgabe wird ferner bei einem chirurgischen Navigationssystem der eingangs beschriebenen Art erfindungsgemäß dadurch gelöst, dass das mindestens eine Markerelement am Grundkörper beweglich gelagert ist, dass die Referenzierungseinheit eine erste Schnittstelleneinrichtung umfasst, welche ausgebildet ist zum in Eingriff Bringen mit einer zweiten, an einem chirurgischen Instrument oder einem Implantat vorgesehenen Schnittstelleneinrichtung in einer Verbindungsstellung, und dass das mindestens eine erste Markerelement in Abhängigkeit der zusammenwirkenden ersten und zweiten Schnittstelleneinrichtungen in eine vordefinierte Markerelementposition bringbar ist.

Ein solches chirurgisches Navigationssystem erleichtert die Arbeit im Operationssaal, da durch die besondere Ausgestaltung der Instrumente, Implantate und Referenzierungseinheiten sowie daran angepasste Funktionalität beziehungsweise Ausbildung des Navigationssystems sowohl eine Position und/oder Orientierung eines Instruments oder Implantats bestimmbar ist, gleichzeitig beziehungsweise darüber hinaus aber auch der Typ des Implantats oder Instruments, und zwar automatisch mittels des Navigationssystems. Dieses umfasst vorzugsweise eine Datenbank aller zur Verfügung stehender Instrumente und Implantate mit zweiten Schnittstelleneinrichtungen und deren Kodierungen, so dass aus einer bestimmten räumlichen Anordnung der Markerelemente der Referenzierungseinheit nach Verbinden mit dem jeweiligen Instrument oder Implantat das Instrument selbst oder zumindest dessen Typ, ebenso bei den Implantaten, automatisch durch das chirurgische Navigationssystem ermittelbar ist. Eine manuelle Zuordnung einer bestimmten Markerelementgeometrie der Referenzierungseinheit zu einem Implantat oder einem Instrument ist auf Grund der erfindungsgemäßen Lösung nicht mehr erforderlich.

Günstigerweise umfasst das chirurgische Navigationssystem eine oder mehrere der oben beschriebenen chirurgischen Referenzierungseinheiten. Die im Zusammenhang mit den bevorzugten Ausführungsformen der Referenzierungseinheiten beschriebenen Vorteile weist dann auch das chirurgische Navigationssystem als Ganzes entsprechend auf.

Des Weiteren ist es günstig, wenn das chirurgische Navigationssystem eines oder mehrere der oben beschriebenen chirurgischen Instrumente umfasst. Die im Zusammenhang mit den bevorzugten Ausführungsformen der Instrumente beschriebenen Vorteile weist dann das chirurgische Navigationssystem in gleicher Weise auf.

Vorzugsweise umfasst das chirurgische Navigationssystem zwei oder mehr Instrumente der oben beschriebenen Art, welche unterschiedliche, kodierte zweite Schnittstelleneinrichtungen aufweisen zur unterschiedlichen Positionierung des mindestens einen Markerelements der Referenzierungseinheit. Werden derartige Instrumente mit einer der oben beschriebenen Referenzierungseinheiten verbunden, wird eine Geometrie der Markerelemente, das heißt insbesondere deren relative Positionen zueinander, in gezielter Weise so geändert, dass das Navigationssystem aus der Bestimmung der Positionen der Markerelemente der Referenzierungseinheit, beispielsweise im Vergleich mit in einer Datenbank hinterlegten Markerelementgeometrien, das mit der Referenzierungseinheit verbundene Instrument oder Implantat automatisch ermitteln kann.

Die nachfolgende Beschreibung bevorzugter Ausführungsformen der Erfindung dient im Zusammenhang mit der Zeichnung der näheren Erläuterung, Es zeigen:
- Figur 1:: eine schematische Gesamtdarstellung eines chirurgischen Navigationssystems;
- Figur 2:: eine teilweise geschnittene Seitenansicht einer in Figur 1 an einem Instrument festgelegten chirurgischen Referenzierungseinheit;
- Figur 3:: eine Ansicht der Referenzierungseinheit aus Figur 2 in Richtung des Pfeils A;
- Figur 4:: eine teilweise geschnittene Ansicht eines ersten Ausführungsbeispiels einer chirurgischen Referenzierungseinheit; und
- Figur 5:: eine Ansicht entsprechend Figur 4 eines zweiten Ausführungsbeispiels einer chirurgischen Referenzierungseinheit.

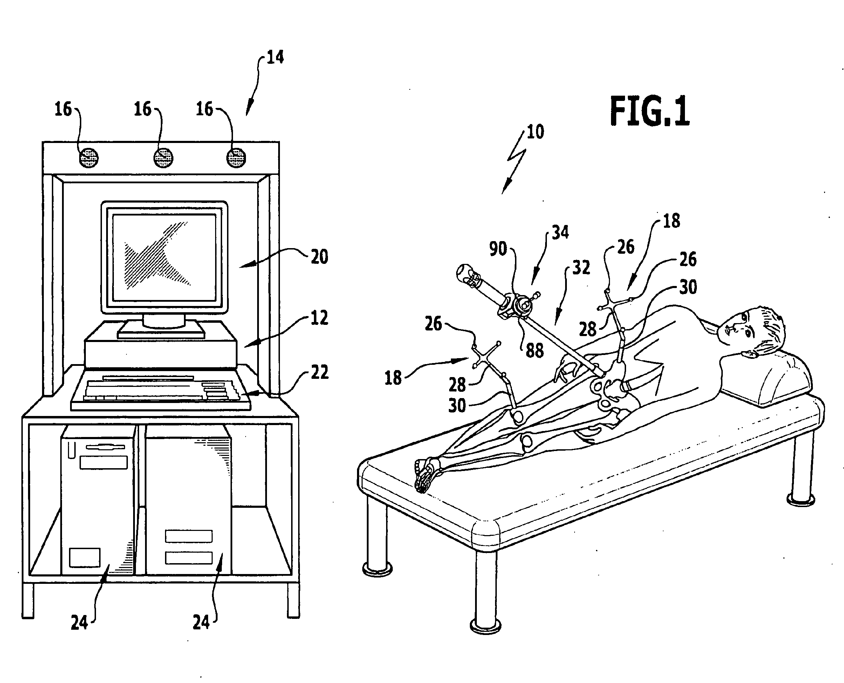

In Figur 1 ist ein insgesamt mit dem Bezugszeichen 10 versehenes chirurgisches Navigationssystem dargestellt, welches mit allen weiteren nachfolgend beschriebenen Vorrichtungen und Teilen insgesamt ein chirurgisches System bildet. Es weist eine von einem als Datenverarbeitungseinrichtung dienenden Computer 12 gesteuerte Sende- und Empfangsstation 14 auf, welche mehrere Sende- und Empfangseinheiten 16 umfasst zum Aussenden und Empfangen von elektromagnetischer Strahlung, sowohl im sichtbaren wie auch im Infrarotbereich, oder von Ultraschall, die von einem Referenzelement 18 ausgesandt oder reflektiert werden. Das chirurgische System und/oder das Navigationssystem 10 umfassen ferner eine Anzeigevorrichtung in Form eines Bildschirms 20 sowie ein Eingabegerät in Form einer Tastatur 22. Zur Erhöhung der Leistungsfähigkeit des chirurgisches Systems beziehungsweise des Navigationssystems 10 können weitere Recheneinheiten 24 mit dem Computer 12 zusammenwirken. Um die Funktion des Navigationssystems 10 in optimaler Weise zu gewährleisten, sind vorzugsweise zwei voneinander räumlich beabstandete Sende- und Empfangseinheiten 16 vorgesehen, es könnten jedoch auch drei oder mehr sein.

Das eine Referenzierungseinheit bildende Referenzelement 18, wie es in Figur 1 dargestellt ist, ist aus dem Stand der Technik bekannt. Es weist mindestens drei passive oder aktive Markerelemente auf. Passive Markerelemente sind geeignet, elektromagnetische Strahlung oder Ultraschall zu reflektieren oder auch verzögert abzustrahlen, die von den Sende- und Empfangseinheiten 16 ausgesandt werden. Aktive Markerelemente 26 dagegen können selbst elektromagnetische Strahlung erzeugen und aussenden, alternativ auch Ultraschall, die beziehungsweise der von den Sende- und Empfangseinheiten 16 empfangen werden können. Werden nur aktive Markerelemente 26 verwendet, können anstelle der Sende- und Empfangseinheiten 16 auch reine Empfangseinheiten vorgesehen werden.

Das Referenzelement 18 umfasst einen kreuzförmigen Träger 28, an dem die Markerelemente 26 in Form von passiven Markerelementen unbeweglich gehalten sind. Die Referenzelemente 18 können an Implantaten, wie beispielsweise den in Figur 1 dargestellten Knochenschrauben 30, oder auch an chirurgischen Instrumenten 32 angeordnet werden.

Sind die beim chirurgischen Eingriff genutzten Referenzelemente 18 identisch ausgebildet, dann können sie nicht automatisch vom Navigationssystem 10 unterschieden werden. Es bedarf daher vor der Durchführung eines chirurgischen Eingriffs unter Verwendung der Referenzelemente 18 einer initialen Positionsbestimmung, welche manuell durchgeführt werden muss. Üblicherweise wird dazu das zu initialisierende Referenzelement 18 mit einem Kalibrierinstrument, dessen Position und Orientierung im Raum bekannt ist, an dafür entsprechend vorgesehenen Punkten palpiert. In Folge der Durchführung dieses Initialisierungsprozesses wird so quasi die Orts- und/oder Orientierungsinformation des Kalibrierinstruments auf das Referenzelement 18 übertragen oder ihm zugeordnet.

Statt der Referenzelemente 18 kann zur Bestimmung einer Position und/oder einer Orientierung beispielsweise des chirurgischen Instruments 32 auch eine erfindungsgemäße Referenzierungseinheit 34 verwendet werden. Sie umfasst einen Grundkörper 36 oder Träger, an welchem mindestens ein Markerelement 38, bei dem in den Figuren 1 bis 3 dargestellten Ausführungsbeispiel insgesamt 4 Markerelemente 38, angeordnet ist. Denkbar wäre es auch, zwei, drei oder aber auch fünf oder mehr Markerelemente 38 vorzusehen. Die Markerelemente 38 sind am Grundkörper 36 beweglich gelagert. Die bewegliche Lagerung der Markerelemente 38 am Grundkörper 36 ist für alle Markerelemente 38 im Prinzip identisch und wird nachfolgend für ein Markerelement exemplarisch beschrieben.

In einer senkrecht zu einer ebenen Grundfläche 40 des im Wesentlichen zylindrischen Grundkörpers 36 verlaufenden Bohrung 42 ist eine langgestreckte Hülse 44 montiert, die ein mit der Grundfläche 40 abschließendes Ende 46 und ein von einer in etwa halbkugeligen, der Grundfläche 40 abgewandten Stirnfläche beziehungsweise Oberseite 48 des Grundkörpers 36 abstehendes zweites Ende 50 aufweist. Die Hülse 44 steht auf etwas mehr als der Hälfte ihrer Gesamtlänge über die Oberseite 48 vor, wobei ein zum Ende 50 hin gerichteter Endabschnitt 52 eine Längsachse 53 definiert, welche mit einer von der Bohrung 42 definierten Längsachse 56 einen Winkel 54 von etwa 35° einschließt.

Das passive Markerelement 38, welches in Form einer Kugel mit einer elektromagnetische Strahlung und/oder Ultraschall reflektierenden Beschichtung auf seiner äußeren Oberfläche versehen ist, ist an einem zylindrischen Führungskörper 58 befestigt, welcher in einer Grundstellung der Referenzierungseinheit 58 ausgehend vom Ende 50 nahezu vollständig in die Hülse 44 eintaucht.

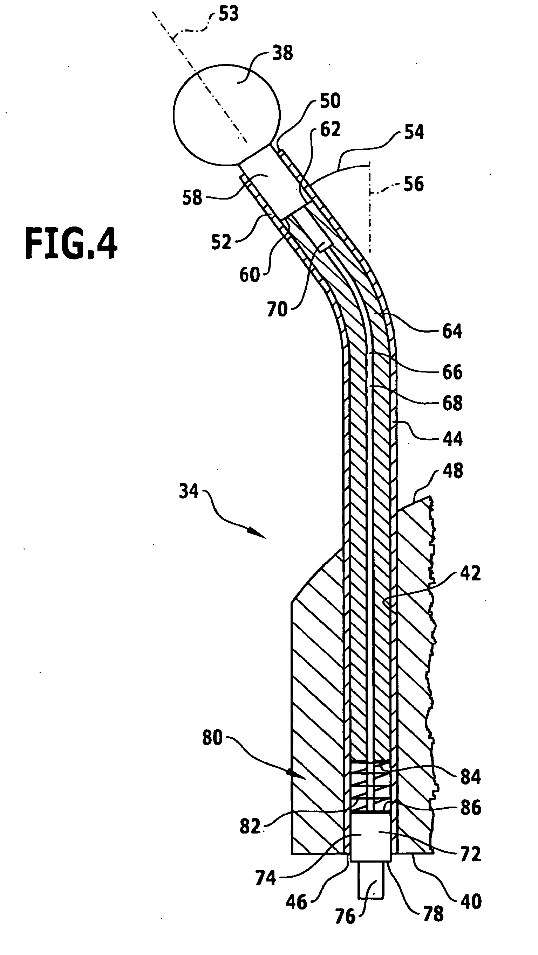

In der in Figur 4 dargestellten Grundstellung liegt eine in die Hülse 44 hineinweisende ebene Fläche 60 des Führungskörpers 58 an einer ringförmigen, aus der Hülse 44 hinausweisenden Endfläche 62 eines den inneren Querschnitt der Hülse 44 ausfüllenden Füllkörpers 64 an, welcher sich bis nahe an das Ende 46 der Hülse 44 in deren Inneren erstreckt. Im Füllkörper 64 ist ein Kanal 66 ausgebildet zur Führung eines stabförmigen Kraftübertragungsglieds 68, welches eine gewisse Elastizität aufweist, um in dem koaxial zu einer Mittellinie der gekrümmten Hülse 44 ausgebildeten Kanal 66 verschiebbar zu sein. Ein Durchmesser des Kanals 66 beträgt etwa nur ein Sechstel eines Innendurchmessers der Hülse 44.

Das Kraftübertragungsglied 68 ist an seinem dem Ende 50 zugewandten Ende mit einem im Durchmesser gegenüber dem Führungskörper 58 kleineren zylindrischen Abschnitt 70 verbunden, das andere Ende des Abschnitts 70 ist unbeweglich an der Endfläche 60 festgelegt.

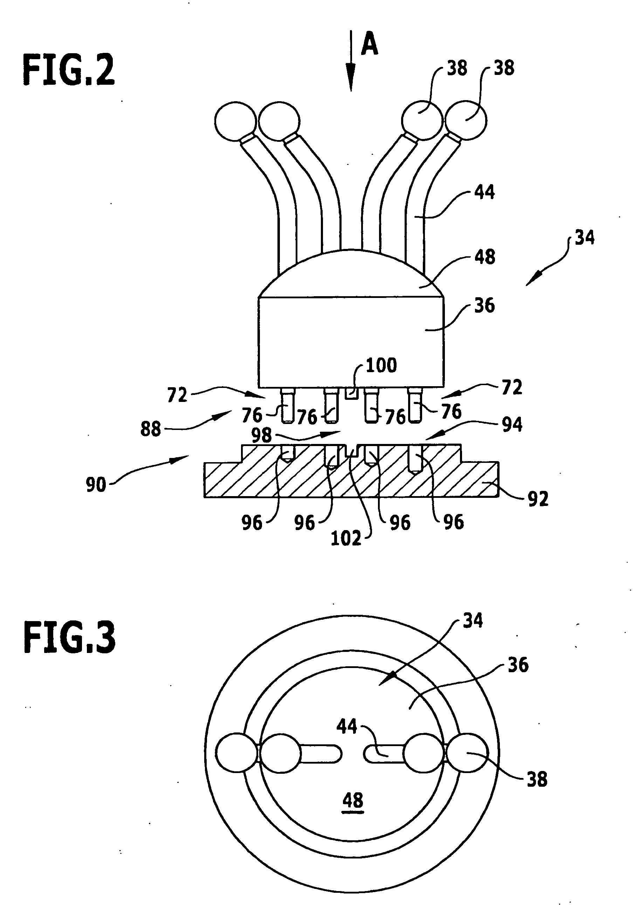

Das in Richtung auf das Ende 46 hin weisende Ende des Kraftübertragungsglieds 68 ist mit einem Schnittstellenglied 72 verbunden, welches einen ersten zylindrischen Abschnitt 74 aufweist, dessen Außendurchmesser an den Innendurchmesser der Hülse 44 angepasst ist, so dass der Abschnitt 74 im Bereich des Endes 46 der Hülse 44 verschiebbar geführt ist. Die Hülse 44 bildet so zumindest einen Teil einer Schnittstellengliedführungseinrichtung. Ein zweiter zylindrischer Betätigungsabschnitt 76 ist koaxial zum Abschnitt 74 auf dessen über die Grundfläche 40 vorstehender Stirnfläche 78 befestigt und ragt in einer Grundstellung der Referenzierungseinheit 34, wie sie in Figur 2 dargestellt ist, vollständig aus dem Ende 46 der Hülse 44 vor. Ein Teil eines eine Rückstelleinrichtung 80 bildenden Rückstellglieds 82 in Form einer Schraubenfeder stützt sich einerseits an einer in Richtung auf das Ende 46 hin weisenden Ringfläche 84 des Füllkörpers 64 ab und umgibt das Kraftübertragungsglied 68. Das andere Ende der Schraubenfeder stützt sich an einer Stirnfläche 86 des Abschnitts 74 ab, welche in Richtung auf die Ringfläche 84 hin weist. Das Kraftübertragungsglied 68 ist fest mit der Stirnfläche 86 verbunden.

Aufgrund der beschriebenen Anordnung kann das Markerelement 38 vom Ende 50 weg und parallel zur Längsachse 53 verschoben werden durch Beaufschlagen des Betätigungsabschnittes 76 des Schnittstellenglieds 72 parallel zur Längsachse 56 in Richtung auf den Füllkörper 64 hin. Dabei wird das Rückstellglied 82 komprimiert, so dass eine Bewegung des Markerelements 38 entgegen der Wirkung der Rückstelleinrichtung 80 erfolgt. Ohne Kraftbeaufschlagung zwingt das Rückstellglied 82 das Schnittstellenglied 72 in die Grundstellung, falls es aus dieser ausgelenkt war, in die Grundstellung zurück, ebenso das über das Kraftübertragungsglied 68 mit dem Schnittstellenglied 72 verbundene Markerelement 38.

Die Schnittstellenglieder 72 der Referenzierungseinheit 34 bilden insgesamt eine erste Schnittstelleneinrichtung 88. Sie ermöglicht es, dass alle Markerelemente 38 der Referenzierungseinheit 34 unabhängig voneinander bewegt und in unterschiedliche Markerelementpositionen gebracht werden können. Markerelementpositionen können insbesondere vordefiniert werden durch eine zweite Schnittstelleneinrichtung 90. Diese kann dauerhaft und fest mit dem Instrument 32 oder einem Implantat 30 verbunden oder mit diesen lösbar verbindbar sein. Die lösbare Verbindbarkeit ermöglicht es, Instrumente 32 oder Instrumente 30 wahlweise mit unterschiedlichen zweiten Schnittstelleneinrichtungen 90 auszustatten.

Die zweite Schnittstelleneinrichtung 90 umfasst einen weiteren Grundkörper 92 in Form einer im Wesentlichen flachen Scheibe. Sie umfasst entsprechend der Anzahl der Markerelemente 38 an der Referenzierungseinheit 34 Kodierelemente 94 in Form von Sacklöchern 96 unterschiedlicher Tiefe. Die Sacklöcher 96 sind so angeordnet, dass die Schnittstellenglieder 72 jeweils in ein Sackloch 96 eintauchen können. Abhängig von der Tiefe der Sacklöcher 96 werden die Schnittstellenglieder 72, die mit dem Betätigungsabschnitt 76 an einem Boden der Kodierelemente 94 anstoßen, wenn diese nicht so tief sind wie eine Höhe des durch das Schnittstellenglied 72 gebildeten Vorsprungs, entgegen der Wirkung der Rückstelleinrichtung 80 in die Hülse 44 hineingezwungen, wodurch das mit dem Schnittstellenglied 72 gekuppelte Markerelement 38 vom Ende 50 weg bewegt wird, und zwar soweit, wie das Schnittstellenglied 72 weiter über die Grundfläche 40 vorsteht als das zugehörige Sackloch 96 tief ist.

Durch entsprechende Ausbildung der Sacklöcher 96 können somit eindeutige Markerelementgeometrien definiert werden, das heißt Positionen der Markerelemente 38 der Referenzierungseinheit 34 relativ zueinander im Raum. Sind derartige Markerelementgeometrien in einer Datenbank des Navigationssystems 10 hinterlegt, so kann umgekehrt bei Bestimmung der Positionen der Markerelemente 38 mit dem Navigationssystem 10 die Kodierung der zweiten Schnittstelleneinrichtung 90 ermittelt werden. Ist die zweite Schnittstelleneinrichtung 90 einem bestimmen Instrument 32 zugeordnet, so kann in Folge des Zusammenwirkens der ersten und zweiten Schnittstelleneinrichtungen 88 und 90 das Navigationssystems 10 nicht nur eine Position und/oder Orientierung der Referenzierungseinheit 34 im Raum bestimmen, sondern gleichzeitig auch das mittels der zweiten Schnittstelleneinrichtung 90 kodierte Instrument 32. Eine wie oben beschriebene initiale Kalibrierung der Referenzierungseinheit 34 und Zuordnung zum Instrument 32 durch eine Bedienperson in manueller Weise ist daher nicht mehr erforderlich.

Zum sicheren mechanischen Verbinden des Grundkörpers 36 mit dem Grundkörper 92 kann ferner eine Kupplungseinrichtung 98 vorgesehen sein, welche mindestens ein erstes Kupplungselement 100 an der Referenzierungseinheit 34, beispielsweise am Grundkörper 36, umfasst, sowie ein zweites Kupplungselement 102 an der zweiten Schnittstelleneinrichtung 90, beispielsweise am Grundkörper 92. Das erste Kupplungselement 100 und das zweite Kupplungselement 102 sind vorzugsweise so ausgebildet, dass in einer Verbindungsstellung der ersten und zweiten Schnittstelleneinrichtungen 88 und 90 die Kupplungselemente 100 und 102 kraft- und/oder formschlüssig ineinander greifen. Das erste Kupplungselement 100 kann beispielsweise in Form eines vom Grundkörper 36 abstehenden Vorsprungs ausgebildet sein, das zweite Kupplungselement 102 in Form einer korrespondierend zum ersten Kupplungselement 100 ausgebildeten Ausnehmung, oder aber auch umgekehrt.

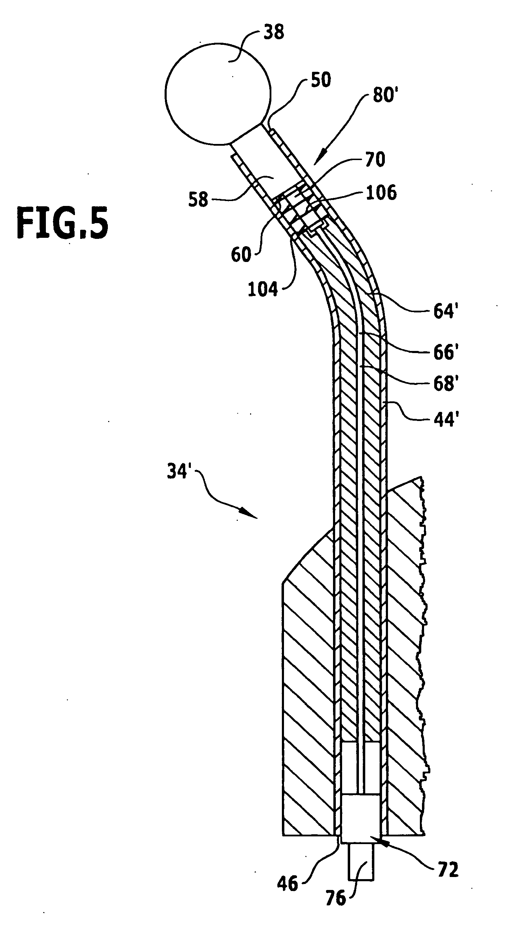

Eine etwas modifizierte Variante des Kraftübertragungsmechanismus zum Übertragen einer Betätigungskraft vom Schnittstellenglied 72 auf das Markerelement 38 ist in Figur 5 dargestellt. Teile der modifiziert ausgebildeten Referenzierungseinheit 34', die auch in der Referenzierungseinheit 34, wie sie in der Figur 4 dargestellt ist, vorgesehen sind, sind mit identischen Bezugszeichen versehen, sollten sie etwas abgewandelt sein mit einem " ' ".

In der Hülse 44' sitzt wie oben beschrieben ein Füllkörper 64' und definiert einen Kanal 66', in dem ein Kraftübertragungsglied 68 analog geführt und an seinem dem Ende 46 der Hülse 44' zugewandten Ende mit einem Schnittstellenglied 72 verbunden ist. Der Füllkörper 64' ragt jedoch nicht so weit bis zum Ende 50 vor wie der Füllkörper 64 bei der Referenzierungseinheit 34. Vielmehr ist eine den Kanal 66' umgebende ringförmige Endfläche 104 ausgebildet, an welcher ein Ende einer ein Rückstellglied bildenden Schraubenfeder 106 befestigt ist. Das andere Ende der Schraubenfeder 106, welche den Abschnitt 70 umgibt, ist fest mit der Endfläche 60 des Führungskörpers 58 verbunden.

Wird der Betätigungsabschnitt 76 in Richtung in die Hülse 44' hinein gedrückt, wird der Führungskörper 58 mit dem Markerelement 38 etwas weiter aus dem Ende 50 der Hülse 44 herausgeschoben. Da die freien Enden der Schraubenfeder 106 fest mit dem Füllkörper 64' beziehungsweise dem Führungskörper 58 verbunden sind, wird die Schraubenfeder 106, anders als das Rückstellglied 82 bei der Referenzierungseinheit 34, auf Zug und nicht auf Druck belastet. Trotzdem wird das Markenelement 38 entgegen der Wirkung einer Rückstelleinrichtung 80' aus der in Figur 5 dargestellten Grundstellung ausgelenkt. Wirkt keine Betätigungskraft mehr auf das Schnittstellenglied 72, dann zieht sich die Schraubenfeder 106 wieder zusammen und überführt das Markerelement 38 wieder in seine Grundstellung.

## Patentansprüche

1. Chirurgische Referenzierungseinheit (34) für ein chirurgisches Navigationssystem (10), mit dem eine Position und/oder Orientierung der Referenzierungseinheit (34) im Raum bestimmbar ist, welche Referenzierungseinheit (34) einen Grundkörper (36) und mindestens ein am Grundkörper (36) angeordnetes Markerelement (38) umfasst, dessen Position im Raum durch das Navigationssystem (10) bestimmbar ist, wobei das mindestens eine Markerelement (38) am Grundkörper (36) beweglich gelagert ist, wobei die Referenzierungseinheit (34) eine erste Schnittstelleneinrichtung (88) umfasst, welche ausgebildet ist zum in Eingriff Bringen mit einer zweiten, an einem chirurgischen Instrument (32) oder einem Implantat (30) vorgesehenen Schnittstelleneinrichtung (90) in einer Verbindungsstellung, **dadurch gekennzeichnet, dass** die erste Schnittstelleneinrichtung derart ausgebildet ist, dass das mindestens eine erste Markerelement (38) in Abhängigkeit der zusammenwirkenden ersten und zweiten Schnittstelleneinrichtungen (88, 90) automatisch in eine vordefinierte Markerelementposition bringbar ist.

2. Chirurgische Referenzierungseinheit nach Anspruch 1, **gekennzeichnet durch** eine erste Kupplungseinrichtung (100), welche ausgebildet ist zum Kuppeln mit einer zweiten, entsprechend ausgebildeten Kupplungseinrichtung (102), welche an einem chirurgischen Instrument (32) oder einem Implantat (30) vorgesehen ist, zum lösbaren Verbinden des Grundkörpers (36) mit dem Instrument (32) oder dem Implantat (30).

3. Chirurgische Referenzierungseinheit nach einem der voranstehenden Ansprüche, **gekennzeichnet durch** eine Rückstelleinrichtung (80) zum Überführen des mindestens einen Markerelements (38) von einer aus einer Grundstellung, welche das mindestens eine Markerelement (38) einnimmt, wenn die erste Schnittstelleneinrichtung (88) mit keiner zweiten Schnittstelleneinrichtung (90) gekoppelt ist, ausgelenkten Markerelementstellung in die Grundstellung zurück.

4. Chirurgische Referenzierungseinheit nach einem der voranstehenden Ansprüche, **dadurch gekennzeichnet, dass** die erste Schnittstelleneinrichtung (88) mindestens ein mit dem mindestens einen Markerelement (38) gekoppeltes Schnittstellenglied (72) umfasst, welches derart angeordnet und beweglich gelagert ist, dass in Folge einer auf das Schnittstellenglied (72) einwirkenden Betätigungskraft das mindestens eine Markerelement (38) von der Grundstellung, welche das mindestens eine Markerelement (38) einnimmt, wenn die erste Schnittstelleneinrichtung (88) mit keiner zweiten Schnittstelleneinrichtung (90) gekoppelt ist, in eine ausgelenkte Markerelementstellung bringbar ist.

5. Chirurgische Referenzierungseinheit nach Anspruch 4, **dadurch gekennzeichnet, dass** jedem Markerelement (38) ein eigenes Schnittstellenglied (72) zugeordnet ist.

6. Chirurgische Referenzierungseinheit nach Anspruch 4 oder 5, **dadurch gekennzeichnet, dass** das Schnittstellenglied (72) an der ersten Schnittstelleneinrichtung (88) verschiebbar gelagert ist.

7. Chirurgische Referenzierungseinheit nach einem der Ansprüche 4 bis 6, **dadurch gekennzeichnet, dass** das Schnittstellenglied (72) aus der Grundstellung entgegen der Wirkung der Rückstelleinrichtung (80) in eine ausgelenkte Schnittstellengliedstellung auslenkbar ist.

8. Chirurgische Referenzierungseinheit nach einem der Ansprüche 4 bis 7, **dadurch gekennzeichnet, dass** das Schnittstellenglied (72) einen in der Grundstellung vom Grundkörper (36) mindestens teilweise abstehenden Betätigungsabschnitt (76) umfasst.

9. Chirurgische Referenzierungseinheit nach einem der Ansprüche 4 bis 8, **gekennzeichnet durch** eine Schnittstellengliedführungseinrichtung (44) zum Führen einer Bewegung des Schnittstellenglieds (72) von der Grundstellung in eine ausgelenkte Schnittstellengliedstellung und/oder zurück.

10. Chirurgische Referenzierungseinheit nach einem der Ansprüche 4 bis 9, **dadurch gekennzeichnet, dass** das Schnittstellenglied (72) mit dem mindestens einen Markerelement (38) über ein Kraftübertragungsglied (68) gekoppelt ist.

11. Chirurgisches Instrument (32) mit einer chirurgischen Referenzierungseinheit (34) für ein chirurgisches Navigationssystem (10), mit welchem Navigationssystem (10) eine Position und/oder Orientierung der Referenzierungseinheit (34) im Raum bestimmbar ist, **dadurch gekennzeichnet, dass** die Referenzierungseinheit (34) in Form einer Referenzierungseinheit nach einem der voranstehenden Ansprüche ausgebildet ist.

12. Chirurgisches Instrument nach Anspruch 11, **dadurch gekennzeichnet, dass** die zweite Schnittstellenreinrichtung (90) mindestens ein Kodierelement (94) umfasst, welches im Zusammenwirken mit der ersten Schnittstellenreinrichtung (88) das mindestens eine Markerelement (38) in die vordefinierte Markerelementposition zwingt.

13. Chirurgisches Instrument nach Anspruch 11 oder 12, **gekennzeichnet durch** eine zweite Kupplungseinrichtung (102), welche ausgebildet ist zum Kuppeln mit einer ersten, entsprechend ausgebildeten Kupplungseinrichtung (100), welche an der Referenzierungseinheit (34) vorgesehen ist, zum lösbaren Verbinden des Grundkörpers (36) mit dem Instrument.

14. Chirurgisches Navigationssystem (10) mit mindestens einer Referenzierungseinheit (34, 38) und mit mindestens einer Nachweisvorrichtung (16) zum Detektieren der Position und/oder Orientierung der Referenzierungseinheit (18, 34) im Raum, **dadurch gekennzeichnet, dass** die Referenzierungseinheit (34) in Form einer Referenzierungseinheit nach einem der Ansprüche 1 bis 10 ausgebildet ist.

15. Chirurgisches Navigationssystem nach Anspruch 14, **gekennzeichnet durch** mindestens ein chirurgisches Instrument (32) nach einem der Ansprüche 11 bis 13.

## Claims

1. Surgical referencing unit (34) for a surgical navigation system (10) with which a spatial position and/or orientation of the referencing unit (34) is determinable, said referencing unit (34) comprising a base body (36) and at least one marker element (38) arranged on the base body (36), and the spatial position of said marker element being determinable by the navigation system (10), wherein the at least one marker element (38) is movably mounted on the base body (36), wherein the referencing unit (34) comprises a first interface device (88) which is configured to be brought into engagement with a second interface device (90) provided on a surgical instrument (32) or an implant (30) in a connected position, **characterized in that** the first interface device is designed such that the at least one first marker element (38) is automatically movable into a predefined marker element position in dependence upon the interacting first and second interface devices (88, 90).

2. Surgical referencing unit in accordance with claim 1, **characterized by** a first coupling device (100) which is configured for coupling with a second correspondingly configured coupling device (102) which is provided on a surgical instrument (32) or an implant (30), for releasably connecting the base body (36) to the instrument (32) or the implant (30).

3. Surgical referencing unit in accordance with any one of the preceding claims, **characterized by** a resetting device (80) for transferring the at least one marker element (38) from a marker element position deflected out of a initial position, which the at least one marker element (38) assumes when the first interface device (88) is not coupled to a second interface device (90), back into the initial position.

4. Surgical referencing unit in accordance with any one of the preceding claims, **characterized in that** the first interface device (88) comprises at least one interface member (72) which is coupled to the at least one marker element (38) and is movably mounted and arranged in such a way that as a result of an actuating force acting on the interface member (72), the at least one marker element (38) is movable from the initial position which the at least one marker element (38) assumes when the first interface device (88) is not coupled to a second interface device (90) into a deflected marker element position.

5. Surgical referencing unit in accordance with claim 4, **characterized in that** each marker element (38) has an interface member (72) of its own associated with it.

6. Surgical referencing unit in accordance with claim 4 or 5, **characterized in that** the interface member (72) is mounted for displacement on the first interface device (88).

7. Surgical referencing unit in accordance with any one of claims 4 to 6, **characterized in that** the interface member (72) is deflectable from the initial position against the action of the resetting device (80) into a deflected interface member position.

8. Surgical referencing unit in accordance with any one of claims 4 to 7, **characterized in that** the interface member (72) comprises an actuating section (76) which, in the initial position, projects at least partially from the base body (36).

9. Surgical referencing unit in accordance with any one of claims 4 to 8, **characterized by** an interface member guiding device (44) for guiding movement of the interface member (72) from the initial position into a deflected interface member position and/or back.

10. Surgical referencing unit in accordance with any one of claims 4 to 9, **characterized in that** the interface member (72) is coupled to the at least one marker element (38) by a force transmitting member (68).

11. Surgical instrument (32) with a surgical referencing unit (34) for a surgical navigation system (10), with which navigation system (10) a spatial position and/or orientation of the reference unit (34) is determinable, **characterized in that** the referencing unit (34) is designed in the form of a referencing unit in accordance with any one of the preceding claims.

12. Surgical instrument in accordance with claim 11, **characterized in that** the second interface device (90) comprises at least one coding element (94) which, in interaction with the first interface device (88), forces the at least one marker element (38) into the predefined marker element position.

13. Surgical instrument in accordance with claim 11 or 12, **characterized by** a second coupling device (102) which is configured for coupling with a first correspondingly configured coupling device (100) which is provided on the referencing unit (34), for releasable connection of the base body (36) to the instrument.

14. Surgical navigation system (10) with at least one referencing unit (34, 38) and with at least one detecting device (16) for detecting the spatial position and/or orientation of the referencing unit (18, 34), **characterized in that** the referencing unit (34) is designed in the form of a referencing unit (34) in accordance with any one of claims 1 to 10.

15. Surgical navigation system in accordance with claim 14, **characterized by** at least one surgical instrument (32) in accordance with any one of claims 11 to 13.

## Revendications

1. Unité de référencement chirurgicale (34) pour un système de navigation chirurgical (10), avec lequel il est possible de déterminer une position et/ou une orientation dans l'espace de l'unité de référencement (34), ladite unité de référencement (34) comprenant un corps de base (36) et au moins un élément marqueur (38) agencé sur le corps de base (36) et dont la position dans l'espace peut être déterminée par le système de navigation (10), ledit au moins un élément marqueur (38) étant monté mobile sur le corps de base (36), et l'unité de référencement (34) comportant un premier dispositif d'interface (88), qui est configuré pour être, dans une position de liaison, amené en prise avec un deuxième dispositif d'interface (90) prévu sur un instrument chirurgical (32) ou sur un implant (30),
**caractérisée en ce que** le premier dispositif d'interface est configuré de manière à ce que ledit au moins un premier élément marqueur (38) puisse être amené automatiquement, en fonction des premier et deuxième dispositifs d'interface (88, 90) coopérant mutuellement, dans une position prédéfinie d'élément de marqueur.

2. Unité de référencement chirurgicale selon la revendication 1, **caractérisée par** un premier dispositif d'accouplement (100) configuré pour le couplage avec un deuxième dispositif d'accouplement (102) de configuration correspondante, qui est prévu sur un instrument chirurgical (32) ou sur un implant (30), pour assurer la liaison amovible du corps de base (36) avec l'instrument (32) ou l'implant (30).

3. Unité de référencement chirurgicale selon l'une des revendications précédentes, **caractérisée par** un dispositif de rappel (80) pour transférer ledit au moins un élément marqueur (38) d'une position d'élément de marqueur déplacée par rapport à une position de base, que prend ledit au moins un élément marqueur (38) lorsque le premier dispositif d'interface (88) n'est couplé à aucun deuxième dispositif d'interface (90), en retour à la position de base.

4. Unité de référencement chirurgicale selon l'une des revendications précédentes, **caractérisée en ce que** le premier dispositif d'interface (88) comprend au moins un organe d'interface (72) couplé au dit au moins un élément marqueur (38) et qui est agencé et monté mobile de manière telle que, suite à une force d'actionnement agissant sur l'organe d'interface (72), ledit au moins un élément marqueur (38) puisse être amené de la position de base, que prend ledit au moins un élément marqueur (38) lorsque le premier dispositif d'interface (88) n'est couplé à aucun deuxième dispositif d'interface (90), à une position d'élément de marqueur déplacée.

5. Unité de référencement chirurgicale selon la revendication 4, **caractérisée en ce qu'**à chaque élément marqueur (38) est associé un organe d'interface (72) qui lui est propre.

6. Unité de référencement chirurgicale selon la revendication 4 ou la revendication 5, **caractérisée en ce que** l'organe d'interface (72) est monté coulissant au niveau du premier dispositif d'interface (88).

7. Unité de référencement chirurgicale selon l'une des revendications 4 à 6, **caractérisée en ce que** l'organe d'interface (72) peut être déplacé hors de la position de base, à l'encontre de l'action du dispositif de rappel (80), dans une position déplacée d'organe d'interface.

8. Unité de référencement chirurgicale selon l'une des revendications 4 à 7, **caractérisée en ce que** l'organe d'interface (72) présente un tronçon d'actionnement (76) faisant au moins partiellement saillie du corps de base (36), dans la position de base.

9. Unité de référencement chirurgicale selon l'une des revendications 4 à 8, **caractérisée par** un dispositif de guidage d'organe d'interface (44) pour guider un mouvement de l'organe d'interface (72) de la position de base à une position d'organe d'interface déplacée et/ou en retour.

10. Unité de référencement chirurgicale selon l'une des revendications 4 à 9, **caractérisée en ce que** l'organe d'interface (72) est couplé avec ledit au moins un élément marqueur (38) par l'intermédiaire d'un organe de transmission de force (68).

11. Instrument chirurgical (32) avec une unité de référencement (34) pour un système de navigation chirurgical (10), une position et/ou une orientation dans l'espace de l'unité de référencement (34) pouvant être déterminée à l'aide dudit système de navigation (10), **caractérisé en ce que** l'unité de référencement (34) est configurée sous la forme d'une unité de référencement selon l'une des revendications précédentes.

12. Instrument chirurgical selon la revendication 11, **caractérisé en ce que** le deuxième dispositif d'interface (90) comprend au moins un élément de codage (94), qui, par interaction avec le premier dispositif d'interface (88), force ledit au moins un élément marqueur (38) dans la position d'élément de marqueur prédéfinie.

13. Instrument chirurgical selon la revendication 11 ou la revendication 12, **caractérisé par** un deuxième dispositif d'accouplement (102) configuré pour le couplage avec un premier dispositif d'accouplement (100) de configuration correspondante, qui est prévu sur l'unité de référencement (34), en vue d'assurer la liaison amovible du corps de base (36) avec l'instrument.

14. Système de navigation chirurgical (10) comprenant au moins une unité de référencement (34, 38) et au moins un dispositif d'identification (16) pour détecter la position et/ou l'orientation dans l'espace de l'unité de référencement (18, 34), **caractérisé en ce que** l'unité de référencement (34) est configurée sous la forme d'une unité de référencement selon l'une des revendications 1 à 10.

15. Système de navigation chirurgical selon la revendication 14, **caractérisé par** au moins un instrument chirurgical (32) selon l'une des revendications 11 à 13.
